# EUROPEAN PATENT APPLICATION

(11) **EP 0 751 384 A2**
(43) Date of publication of application: **02.01.1997**
(21) Application number: 96304725.3
(22) Date of filing: 26.06.1996
(51) Int. Cl.: G01L 7/02

(54) **Indicators for external pressure applied to a flexible liquid chamber**

(30) Priority: 30.06.1995 GB 9513323
(71) Applicant: TALLEY GROUP LIMITED, Romsey, Hampshire SO51 9AQ (GB)
(72) Inventor: Evans, Ronald James Patrick, Dorset BH23 4EJ (GB)
(74) Representative: Lomas, Geoffrey Michael

(57) **Abstract**

With reference to Figure 1, an applied pressure indicator 1 (shown in plan view) comprises a hollow, flexible, closed chamber 2 containing a liquid 3 within its interior 4, a liquid outlet duct 5 extending from the chamber interior 4, and means comprising restrictors 6, 7 for restricting outward passage of liquid 3 within the duct 5. When the flexible chamber 2 is subjected to external pressure, as indicated by arrows 8, the volume of liquid 3 displaced from the chamber 2 into the duct 5 is indicative of the intensity of said pressure. The indicator 1 illustrated is a one-piece component of flexible plastics material, in this example a transparent polyethylene. The liquid is a glycerol gel.

## Description

This invention relates to applied pressure indicators and has particular application to interface pressure sensing in the field of medicine.

Interface pressure measuring in medical terms is a practice for measuring pressures on a patient's skin. For example, when the patient is sitting or lying down on a mattress or cushion, pressure being measured of the interface between the supporting surface and the body's skeletal frame. The interface is known as the orthotic interface.

An electromechanical micro-processor controlled unit is often used to measure patient orthotic interface. However, such a unit is expensive.

The present invention provides an inexpensive device which can be used, not only to measure patient orthotic interface, but has other applications. For example, to measure the pressure applied to the limb of a patient when bandaging the limb.

According to the invention, an applied pressure indicator comprises a hollow, flexible, chamber containing liquid, a liquid outlet duct extending from the chamber interior, and means for restricting outward passage of liquid within the duct, whereby, when the chamber is subjected to an external pressure, the volume of liquid displaced from the chamber is indicative of the intensity of said pressure.

At least the liquid outlet duct of the indicator is preferably of transparent material.

The means for restricting outward passage of liquid within the duct may comprise more than one restriction.

The liquid outlet duct may bear markings indicative of external pressure applied to the chamber.

As used herein, the term 'liquid' includes other flowable media, such as pastes.

The liquid in the chamber may comprise a gel. Where more than one indicator is employed the liquids in the respective chambers of the indicators may differ in density and/or colour.

An embodiment of the invention will now be described by way of example only, with reference to the accompanying drawings, wherein :-
Figure 1 is a plan view, in section, of an applied pressure indicator,
Figure 2 illustrates an arrangement wherein a plurality of indicators are employed,
Figures 3, 4 and 5 illustrate modifications, and
Figure 6 is a side view of the indicator illustrated by Figure 1.

In the figures, like reference numerals refer to like features and components.

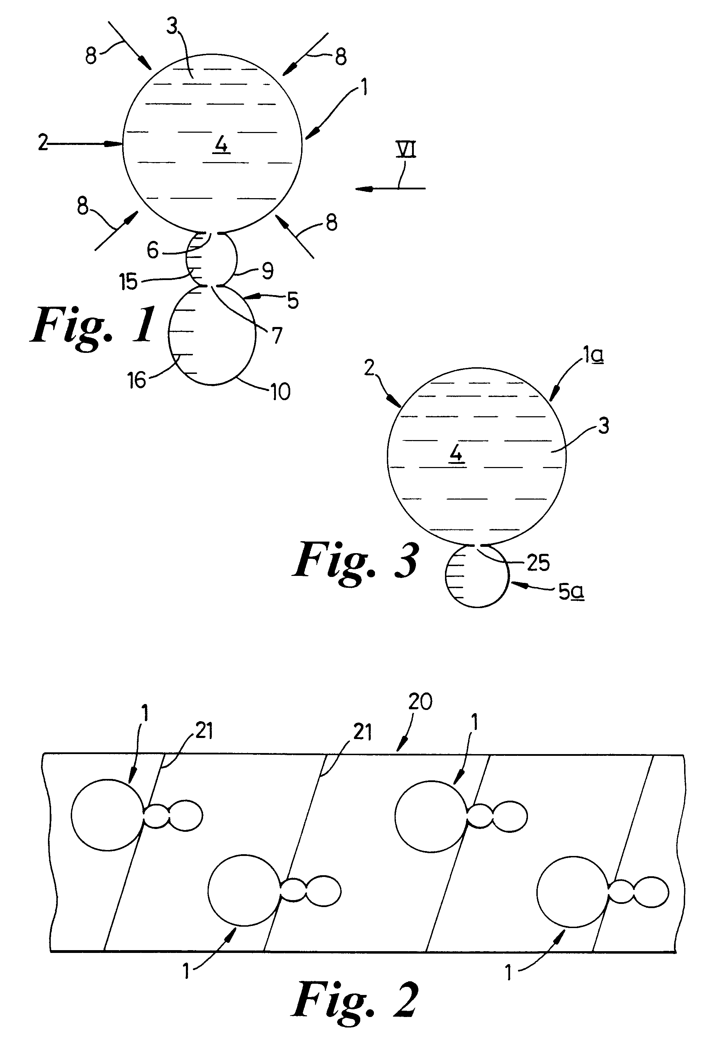

With reference to Figure 1, an applied pressure indicator 1 comprises a hollow, flexible, chamber 2 containing a liquid 3 within its interior 4, a liquid outlet duct 5 extending from the chamber interior 4, and means comprising restrictors 6, 7 for restricting outward passage of liquid 3 within the duct 5.

When the flexible chamber 2 is subjected to external pressure, as indicated by arrow 8, the volume of liquid 3 displaced from the chamber 2 into the duct 5 is indicative of the intensity of said pressure.

The indicator 1 is a one-piece component of flexible plastics material, in this example a transparent polyethylene. The liquid is a glycerol gel.

The duct 5 is blind-ended and is formed by two sections, 9, 10, each of bulbous form. The sections 9, 10 are bulbous to accommodate the displacement of liquid 3. Each bulbous section bears graduation markings, indicated by reference 15 in the case of section 9, and by 16 in the case of section 10. The markings 15, 16 are indicative of the external pressure 8 applied to the flexible chamber 2.

In use, the indicator 1 is positioned where it is desired to measure patient orthotic interface pressure.

The pressure, (arrows 8), applied by the weight of the patient deforms the flexible chamber 2 whereby liquid 3 is displaced into the duct 5, past the restriction 6, and, if substantial enough, past the restrictor 7 as well. The intensity of the applied pressure can then be determined by correlation, that is to say by comparing the extent of liquid displacement with markings 15 or 16. Thus the indicator 1 can be viewed as a form of transducer.

A more approximate indication of intensity of applied external pressure is provided by visual examination of the duct sections 9, 10. If section 9 is full of liquid then the applied pressure is acceptable. If liquid appears in section 10, the applied pressure is too great.

The indicator 1 may also be used to measure pressure applied to the limb of a patient when bandaging the limb.

With reference to Figure 2, a plurality of indicators 1 may be employed, disposed in one or more rows. Such an arrangement has particular advantage in the application of a plaster cast to a patient's leg where it is desirable to monitor a range of pressures.

The indicators 1 of Figure 2 are mounted on a backing 20 comprising a sheet of flexible material.

The lines 21 indicate layers of bandage.

Indicators could be incorporated in bandaging or casting components.

The liquids employed by the indicators 1 of Figure 2 may differ in colour and/or density, in order to suit requirements.

With reference to Figure 3, an indicator la may have an outlet duct 5a employing only one restrictor, namely restrictor 25.

More than two restrictors may be used, if desirable.

Restrictor sizes and/or lengths of outlet duct may vary, according to requirements.

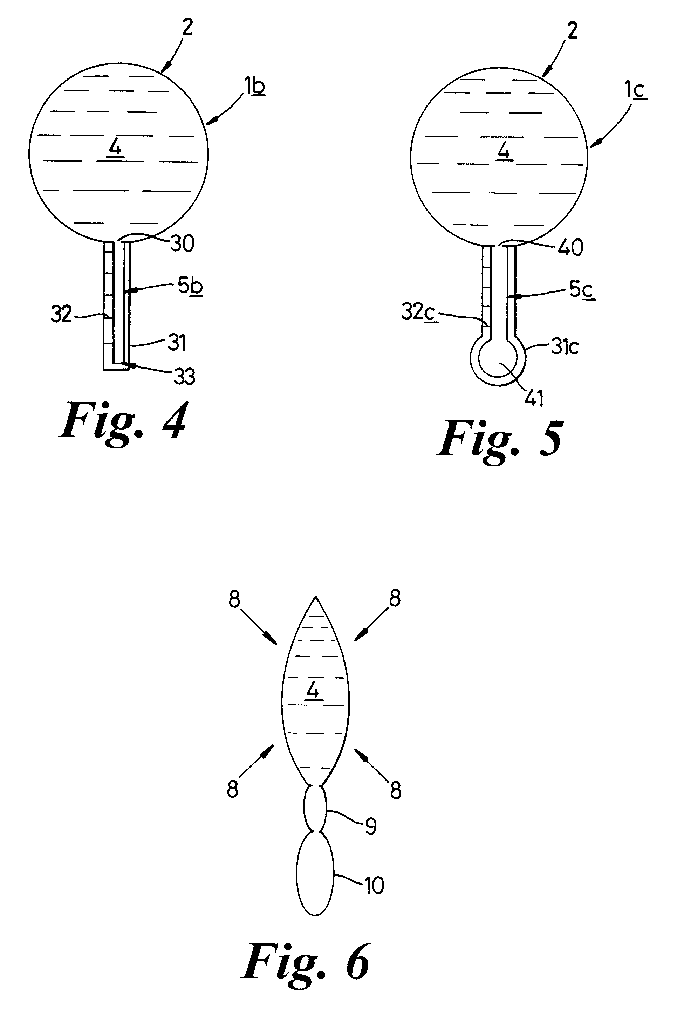

An outlet duct need not be of bulbous form; indeed Figures 4 and 5 illustrate such arrangements.

Figure 4 illustrates an indicator 1b with a single restrictor 30 and a substantially parallel (uniform bore) outlet duct 5b of capillary form. The outlet 5b may be provided with a small-bore air vent 33 at the end remote from chamber 2. A flexible strip or panel 31 attached to outlet 5b bears graduation markings 32. The panel 31 may be integral with the outlet 5a.

Figure 5 illustrates an indicator 1c provided with a single restrictor 40 and an outlet 5c having a bulbous reservoir 41 at the end remote from chamber 2, for accommodating air pushed out of the outlet 5a by displacement of liquid from chamber 2. A graduated panel 31c is mounted on the indicator 1c or is integral therewith,

Where possible and desirable, any of the features or arrangements disclosed herein may be substituted for, or added to, other of said features or arrangements.

Indicators 1, 1a, 1b, 1c may be disposable after use, or may be rendered reusable by displacing, by squeeze action, liquid back into the chambers 2.

## Claims

1. An applied pressure indicator characterised in that it comprises a hollow, flexible, chamber (1, 1a, 1b, 1c) containing liquid (3), a liquid outlet duct (5, 5a, 5b, 5c) extending from the chamber interior (4), and means (6, 7, 25, 40) for restricting outward passage of liquid within the duct, whereby, when the chamber (1, 1a, 1b, 1c) is subjected to an external pressure (8), the volume of liquid (3) displaced from the chamber is indicative of the intensity of said pressure.

2. An applied pressure indicator as claimed in claim 1, wherein at least the liquid outlet duct (5, 5a, 5b, 5, 5d) of the indicator is of transparent material.

3. An applied pressure indicator as claimed in claim 1 or 2, wherein the means (6, 7) for restricting outward passage of liquid within the duct comprise more than one restriction.

4. An applied pressure indicator as claimed in claim 1, 2, or 3, wherein the liquid outlet duct bears markings (15, 16, 32, 32c) indicative of external pressure applied to the chamber.

5. An applied pressure indicator as claimed in any one of claims 1 to 4 wherein the liquid (3) in the chamber (1, 1a, 1b, 1c) comprises a gel.

6. An applied pressure indicator as claimed in claim 5, wherein the gel comprises a glycerol gel.

7. An applied pressure indicator as claimed in any one of claims I to 6, wherein the liquid outlet duct (5, 5a, 9, 10) is of bulbous form.

8. An applied pressure indicator as claimed in any one of claims 1 to 6, wherein the liquid outlet duct is formed by two sections (9, 10), each section being of bulbous form.

9. An applied pressure indicator as claimed in any one of claims 1 to 6, wherein the liquid outlet duct (5b, 5c) is of substantially uniform bore.

10. An applied pressure indicator as claimed in any one of claims 4 to 9, wherein said markings are borne on a panel (31, 31c) attached to the liquid outlet duct.

11. An assembly comprising a plurality of applied pressure indicators, each according to any one of claims 1 to 10, mounted on a backing (20).

12. An assembly as claimed in claim 11, wherein the backing (20) comprises a sheet of flexible material.

13. An assembly as claimed in claim 11 or 12, wherein the liquids employed by the applied pressure indicators differ in colour and/or density.

14. A method of measuring patient orthotic interface pressure, comprising use of an applied pressure indicator as claimed in any one of claims 1 to 10, whereby the weight of the patient displaces liquid (3) from the chamber (1, 1a, 1b, 1c) of the indicator, into the liquid outlet duct thereof, and subsequently conducting a visual examination of the outlet duct in order to obtain an indication of said interface pressure.

15. A method of measuring pressure applied to a limb of a patient when bandaging the limb, comprising incorporating an assembly as claimed in claim 12 in said bandaging, and subsequently conducting a visual examination of the liquid outlet ducts of the indicators of the assembly in order to obtain an indication of pressure applied to the patient's limb by said bandaging.

16. A method of measuring pressure applied to the limb of a patient by a plaster cast, comprising incorporating an assembly as claimed in claim 12 in said cast, and subsequently conducting a visual examination of the liquid outlet ducts of the indicators of the assembly, in order to obtain an indication of pressure applied to the patient's limb by said plaster cast.
